# EUROPEAN PATENT SPECIFICATION

(11) **EP 3 763 304 B1**
(45) Date of publication and mention of the grant of the patent: **10.07.2024**
(21) Application number: 20181574.3
(22) Date of filing: 23.06.2020
(51) Int. Cl.: A61B 17/17, A61B 17/88

(54) **SURGICAL GUIDE FOR DESROTATIONAL OSTEOTOMY OPERATIONS WITH ROTATION OF THE SECTIONS OF THE BONE ON THE CUTTING PLANE**
CHIRURGISCHE FÜHRUNG FÜR DESROTATIONSOSTEOTOMIEOPERATIONEN MIT ROTATION DER KNOCHENABSCHNITTE IN DER SCHNITTEBENE
GUIDE CHIRURGICAL POUR DES OPÉRATIONS D'OSTÉOTOMIE DE DÉROTATION AVEC ROTATION DES SECTIONS DE L'OS SUR LE PLAN DE COUPE

(30) Priority: 09.07.2019 ES 201930636
(43) Date of publication of application: 13.01.2021
(73) Proprietor: Saúde Atlântica - Gestão Hospitalar, S.A., Porto (PT)
(72) Inventor: SANCHEZ ALVAREZ, Jose Miguel, 01007 VITORIA-GASTEIZ (ES); SANCHEZ ARIZMENDIARRIETA, Xabier, 01007 VITORIA-GASTEIZ (ES); FIZ SANCHEZ, Juan Nicolas, 01004 VITORIA-GASTEIZ (ES)
(74) Representative: Couto, Cláudia

(56) References cited:
- WO-A1-2017/070318
- WO-A1-2019/038240
- WO-A2-2008/005271

## Description

### TECHNICAL FIELD

The present invention relates to a kit including a surgical guide which adapts to the bone in the areas of interest fundamentally participates, equipped with a plurality of protrusions with axial holes wherein respective pairs of parallel Kirschner wires, two by two, are able to be inserted, wires which act as support means for the guide, as well as guides and a retaining stop in the cutting of the bone.

The object of the invention is to provide a kit with a second guide, which can be implanted easily and in a completely precise manner through two of the aforementioned Kirschner wires, such that this guide enables the bone to rotate with respect to the cutting plane made beforehand, to the exact point which has been calculated beforehand, without risks of error in said angular movement, additionally enabling both sections of the bone to be immobilised in the final rotation position thereof in order to facilitate the implantation manoeuvres of the corresponding osteosynthesis plate.

Consequently, the object of the invention is to provide a kit which enables an operation as precise as possible, quick, as well as safe for the surgeon, reducing the dependence on the skill of the surgeon, in other words, minimising possible human errors.

### BACKGROUND OF THE INVENTION

Torsional alterations in the extremities are a frequent condition in the general population. It is common for them to go unnoticed or stay hidden in the diagnostic process; alterations in the frontal or sagittal plane are more evident and therefore easier to diagnose, a rotational component frequently being associated in the deformity. If this component is not diagnosed, it is hard for it to be corrected. Moreover, the relationship between said deformities and the development of osteoarthritis is proven.

The correct analysis of these deformities requires a more extensive radiological study than simple radiology, with computed tomography (CT) usually being chosen. This technique enables reconstructions to be made in the 3 planes of the space, such that the alterations can be measured on a torsional level.

Advances in computer software for image processing enable a complete analysis of the rotational component in each bone. This is especially useful in long bones (such as the femur and tibia in the lower limbs; and the humerus, ulna, and radius in the upper limbs). In this manner, the rotation in each of the studied bones can be calculated exactly. The methods for measuring torsion angles are difficult, and the precision in the measurement of these deformities considerably increases if we use 3D reconstruction techniques.

Moreover, it enables virtual corrections of torsional alterations to be performed, giving information about the final result of a possible surgical operation.

Likewise, it enables surgical guides to be generated for intraoperative use. The guides adapt precisely to the bone surface and guide the correction of the torsion angle at all times. Until now, said correction had to be performed by taking intraoperative visual references, these techniques being highly dependent on the observer and therefore the results of the correction not being very precise. Currently, the systems of surgical guides made in a personalised manner by means of 3D printing prevent any variability depending on the observer and enable the planning made in the virtual model to be reproduced exactly.

Thus, it is a matter of sectioning the affected bone and modifying the relative position between the two sections of the bone obtained, such that they rotate with respect to the sectioning plane, and it may become necessary to vary the angle they form with respect to the frontal or sagittal plane.

In this manner, the osteoarthritis symptoms are alleviated, causing the forces to be distributed correctly by realigning the leg.

Thus, osteotomy techniques are more suitable than arthroplasty techniques, since it is a less invasive technique and provides a good recovery with a lower chance of failure.

In this sense, reference is made to patent application P201830153, published as ES2723549, wherein a sort of kit is described, wherein a surgical guide which adapts to the bone in the areas of interest fundamentally participates, made of a single piece with an elongated configuration that defines a plurality of protrusions with axial holes wherein respective Kirschner wires are able to be inserted, which will be introduced into said guides, with different inclinations, in a certain order, position and depth, such that both the order and the depth of placement are defined beforehand in the Kirschner wires and in the surgical guide itself, such that once the wires have been placed on the bone, the surgical guide is then removed, after which the cutting of the osteotomy is then per- formed, using a surgical cutting saw, such that the intermediate wires act as a guide element for the cutting tool, while the lower Kirschner wires act as a stop in the movement of said cutting tool, the cutting being able to be performed in a single plane, or in a bisector plane, the one formed by both intermediate and lower pairs of Kirschner wires, as required by the specific case in question.

In any case, this system only enables the angulation of the two bone sections to be modified in a single work plane, such that it does not enable the rotation of a section of bone with respect to the other to be carried out in a precise and unequivocal manner on the cutting plane itself in the cases wherein a rotational alteration of the bone is associated which requires a derotational osteotomy.

In patent application WO 2019/038240, a guide is foreseen wherein a series of protrusions with holes for the passage of Kirschner wires for guiding the cutting and stabilization of the guide participates, wherein a lateral groove closed on one of the ends thereof participates which defines a slot through which a Kirschner wire is moveable by means of which a rotation of the section of bone is achieved with respect to the other one on the cutting plane itself in a controlled manner, said wire acting as a stop in the bottom of the lateral groove described. WO 2019/038240 discloses a kit according to the pre-amble of appended independent claim 1.

### DESCRIPTION OF THE INVENTION

In accordance with the present invention, there is provided a kit as defined in appended independent claim 1. Embodiments of the present invention are defined in the appended claims dependent on independent claim 1. The surgical guide for the implantation of osteosynthesis plates in osteotomy operations with bone rotation on the cutting plane proposed by the invention comes to fill the technical gap previously set forth, such that combined with a system of guides of the type described in invention patent P201830153, published as ES2723549, or independently, it enables the possibilities of correcting deviations in osteotomy operations to be increased, even in the three Cartesian planes.

To do so, and more specifically, it has been foreseen that starting from the structure of the guide de- scribed in invention patent P201830153, published as ES2723549, it is complemented by an additional protrusion intended for the passage of a seventh Kirschner wire, the interlocking point of which has been pre-calculated beforehand, in order to determine a stop element in the movement between bone sections starting from the new guide of the invention, as will be seen below.

Thus, once the different Kirschner wires have been implanted with the guide described in invention patent P201830153, published as ES2723549, as well as the aforementioned seventh Kirschner wire, said guide has been removed and the bone has been cut starting from the corresponding Kirschner wires, it has been foreseen that the two intermediate wires and the two lower wires are removed, the two upper wires acting as insertion means for the new guide of the invention, which is made of a personalised element for each patient obtained by means of an additive manufacturing process based on a 3D model, which is generated from a computerised axial tomography (CAT) process or magnetic resonance, carried out in the pre-operative process on the extremity of the bone to be treated, mainly the tibia, having a vertically elongated configuration, with a surface resting on the bone identical to the physical features thereof by virtue of the aforementioned process.

This guide ends in correspondence with the lower extremity thereof with a lateral groove, elongated and open on one of the ends thereof, which in the initial implantation position of the guide is arranged facing the seventh Kirschner wire, such that when the rotation of the lower section of the bone is performed on the cutting plane, the seventh Kirschner wire will move together with said lower section through the groove of the guide, to a point wherein the mentioned seventh Kirschner wire stops against the bottom of the lateral groove of the guide, a point which will correspond to the exact pre-calculated deviation point foreseen.

Depending on the cutting plane that is foreseen, whether it is simply horizontal, determined by the pair of intermediate wires, using two cutting planes in order to remove a wedge or bisector plane of the osteotomy, by means of the pairs of intermediate and lower wires, or by using a single oblique cutting plane, deviations in the three Cartesian planes can be corrected.

According to a preferred variant embodiment of the invention, the new guide will be able to be equipped in the upper area thereof with a pair of protrusions, having respective axial and through holes, acting as guides for a pair of Kirschner wires parallel to each other, wires the function of which is to pass through both sections of the bone, in order to temporarily immobilise them, while the osteosynthesis plate is secured, staples are applied or intramedullary nails are applied, such that once any of these solutions have been implanted, said wires will be removed, both the guide and the wires acting as a guide far the latter having been removed beforehand.

Thus, from this structuring, it is not necessary to think about or have to debate the exact location of the guide, the angle to cut or the angle to move one section of bone with respect to the other, since the Kirschner wires used determine it, almost completely reducing the risk of human error in the placement of the guide and calibration of the same since this guide does not require calibration during the operation. This implies that with the help of the 3D models obtained in the preoperative period and the preoperative calculations supported by the computer tools, we were able to obtain a personalised osteotomy guide for each patient by means of additive manufacturing, which considerably improves the degree of accuracy of the operation and significantly reduces the time of the surgical operation, with all that it entails: less probability of infection, less time for anaesthesia, less ischaemia time and more precise surgery.

Finally, it is worth noting the fact that with the guide system of the invention, since all the parameters are pre-calculated by a computer, it is not necessary to continuously supervise the work being performed by means of X-ray equipment, unnecessarily radiating the surgeon's hands.

### DESCRIPTION OF THE DRAWINGS

As a complement to the description provided below, and for the purpose of helping to make the features of the invention more readily understandable, in accordance with a practical preferred embodiment thereof, said description is accompanied by a set of plans which, by way of illustration and not limitation, represent the following:

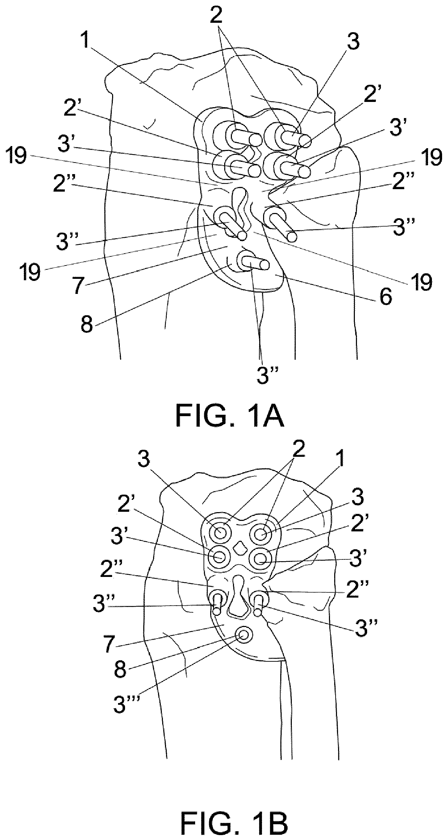

Figures 1A and 1B show different perspective views of a surgical guide far osteotomy operations, specifically a variant of the guide described in invention patent P201830153, published as ES2723549, showing how the guide includes a seventh protrusion with the corresponding axial hale thereof for the exact implantation of a seventh Kirschner wire, acting as a rotation stop for the guide object of the invention.

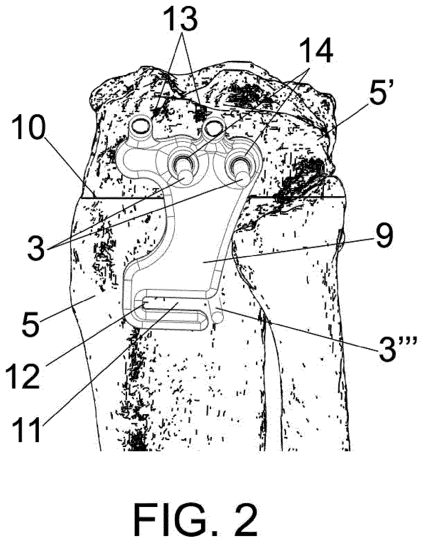

Figure 2 shows a front perspective view of the bone once the cutting of same has been performed by means of the Kirschner wires implanted through the guide of figure 1, having removed the pairs of intermediate and lower wires, the upper wires acting as means far guiding and implanting the new guide, the lower lateral groove of which in said initial position is facing the seventh Kirschner wire implanted by means of the guide of figure 1.

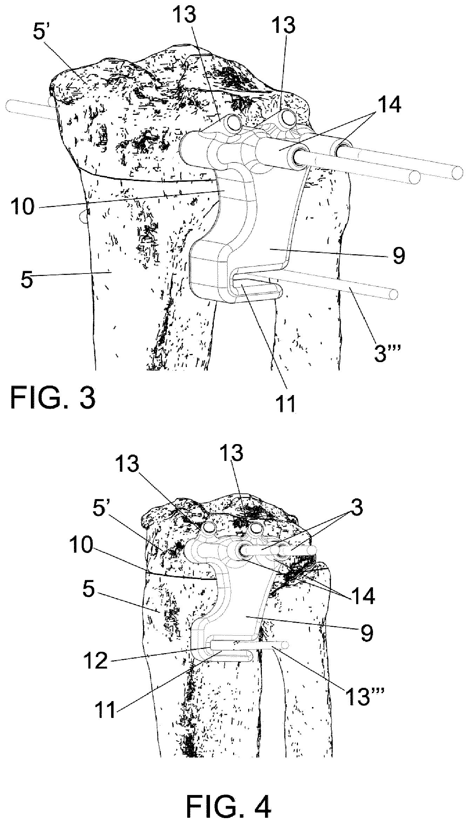

Figures 3 and 4 show corresponding views in different perspectives of the assembly of figure 2.

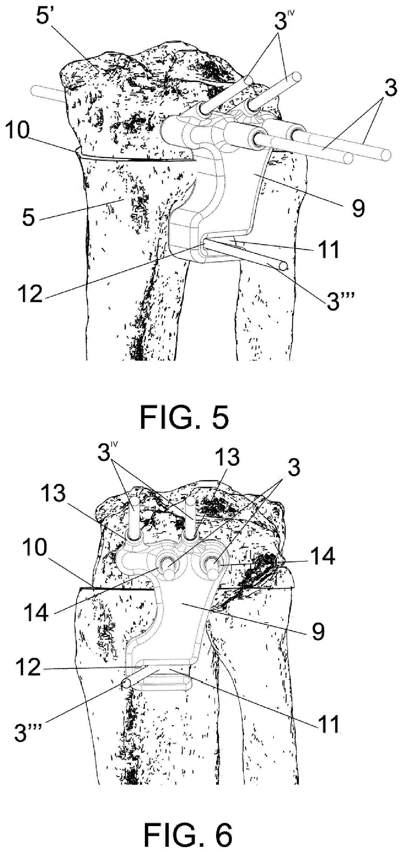

Figures 5 and 6 show corresponding views similar to those of figures 4 and 2, but wherein the lower bone section has been rotated with respect to the upper section, until reaching the exact correction point determined by the stop made by the bottom of the groove of the guide on the mentioned seventh wire.

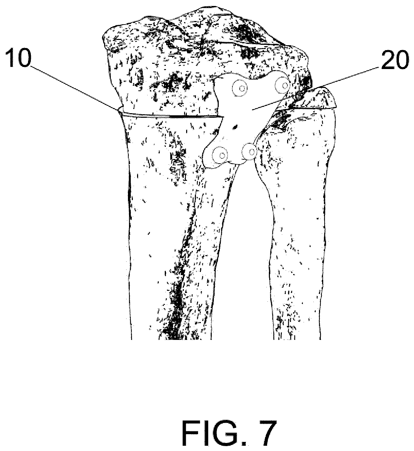

Figure 7 finally shows a perspective view wherein the duly implanted osteosynthesis plate finally appears once the final relative rotation position between the bone sections has been reached.

### PREFERRED EMBODIMENT OF THE INVENTION

In light of the outlined figures, and especially of figure 1, it can be seen how the invention represents an improvement with respect to the assembly described in invention patent P201830153, published as ES2723549, wherein a guide (1) participates, which is structured in a single piece with an elongated configuration which defines a plurality of protrusions (2-2'-2")with axial holes in order for each of them to house a Kirschner wire (3-3'-3"), and weakened areas (19) wherein it will break with the surgical chisel in the corresponding phase of the method; protrusions (2) which are distributed in three pairs of alignments, two upper ones, two intermediate ones and two lower ones.

The guide (1) is obtained by means of an addi-tive manufacturing process starting from a 3D model, which is generated from computerised axial tomography (CAT) or magnetic resonance, carried out in the pre-operative process on the extremity of the bone (5) to be treated, having a vertically elongated configuration, with a surface resting on the bone complementary to the physical features thereof by virtue of said preoperative process.

The upper protrusions (2) enable a pair of upper parallel Kirschner wires (3) to be guided, the function of which is to maintain the osteotomy guide (1) in the suitable and pre-calculated position during the operation.

The intermediate protrusions (2') enable a second pair of intermediate parallel Kirschner wires (3') to be guided, acting as guide means for the cutting tool in the upper plane of the bisector plane of the osteotomy.

Moreover, the lower protrusions (2") enable a pair of parallel lower wires (3") to be guided, intended to act as a stop for the cutting tool when the upper cutting plane is being obtained.

Optionally, the guide in turn ends on the bottom in a lateral bend or arm (6) which acts as positioning and stabilising means for the entire assembly of the guide.

Therefore, according to the invention, it has been foreseen that the guide (1) includes, on the lower sector (7) thereof, a protrusion (8) with the corresponding axial hole thereof for the passage of a seventh Kirschner wire (3‴), the interlocking point of which has been pre-calculated beforehand, in order to determine a stop element in the movement between bone sections (5-5') starting from the new guide (9) of the invention, the one shown in figures 2 to 6.

Thus, once the different wires have been implanted with the guide described in invention patent P201830153, published as ES2723549, as well as the mentioned seventh wire, said guide has been removed and the cutting (10) of the bone has been performed starting from the corresponding wires (3' and 3"), it has been foreseen that the two intermediate wires (3') and the two lower wires (3") are removed, the two upper wires (3) acting as insertion means far the new guide (9) of the invention through a pair of upper protrusions (14), equipped with through holes for said wires.

This new guide (9) is made of an element personalised for each patient obtained through an additive manufacturing process starting from a 3D model, which is generated starting from computed axial tomography (CAT) or magnetic resonance, such that in the lower extremity thereof a lateral groove (11) is established, elongated and open on one of the ends thereof, which in the initial implantation position thereof of the guide is arranged facing the seventh Kirschner wire (3‴), such that when the rotation of the lower section (5) of the bone is carried out on the cutting plane (10), the seventh wire (3"') will be moved together with said lower section (5), to a point wherein the bottom (12) of said lateral groove (11) stops against the seventh Kirschner wires (3‴), said position corresponding to the exact pre-calculated correction position.

The guide (9) may have a pair of additional protrusions (13) on top with axial through hales, for guiding a fourth pair of Kirschner wires (3^{iv}) parallel to each other, wires the function of which is to cross through both sections of the bone (5'-5) as shown in figures 5 and 6, once both sections of the bone (5'-5) are in the correction position thereof, such that once they have been implanted, the rest of the wires will be removed, as well as the guide itself, temporarily immobilising both sections, while the osteosynthesis plate (20) is secured, staples are applied or intramedullary nails are applied, after which said Kirschner wires (3^{iv}) will be finally removed, closing the wound made, with the approach used in the usual surgical methods.

Lastly, it only remains to point out that although the present practical exemplary embodiment, as well as the section of the description of the invention, has been described by virtue of the solution with which maximum precision is achieved for the purpose of the invention, the applicant does not rule out the possibility of a simpler solution wherein the device only includes an upper protrusion (13), for the passage of a Kirschner wire, a solution which although it is less stable, is less intrusive, and may be interesting in certain operations.

## Claims

1. A kit comprising:
a first surgical guide (9) for derotational osteotomy operations with rotation on a cutting plane of a lower section (5) of a bone with respect to an upper section (5') of the bone, and
a second guide (1),
the first surgical guide (9) being constituted from a guide body obtained from a personalised 3D model generated by means of computerised axial tomography (CAT) or magnetic resonance of the bone to be operated on, the guide body having an elongated configuration, with a surface resting on the bone complementary to physical features thereof, with the particularity that the mentioned guide body (9) has at least two upper protrusions (14), equipped with through holes, for a pair of upper and parallel Kirschner wires (3) for stabilising the guide on the upper section (5') of the bone, a guide body which has, in correspondence with the lower extremity thereof, a lateral groove (11), elongated and open on one of the ends thereof, said lateral groove (11) acting as a movement guide and as a stop in the angular movement in rotation of the lower section (5) of the bone on the cutting plane (10) with respect to the upper section (5') of the bone for a seventh Kirschner wire (3‴) which can be implanted in the lower section (5) of the bone;
**characterised in that**:
said second guide (1) is obtained in a single piece, with an elongated configuration wherein three pairs of protrusions (2-2'-2") are defined with axial holes in order for each of them to house a pair of parallel Kirschner wires (3-3'-3"), two by two;
the three pairs of protrusions (2-2'-2") of said second guide (1) consisting of:
two upper protrusions (2) for guiding the pair of upper and parallel Kirschner wires (3) for maintaining the osteotomy guide (1) in the suitable position during the operation;
two intermediate protrusions (2') for guiding a second pair of parallel Kirschner wires (3'), said second pair of parallel Kirschner wires (3') configured to act as guide means for the cutting tool when the cutting plane is being obtained during the osteotomy; and
two lower protrusions (2") for guiding a third pair of parallel wires (3"), intended to act as a stop for the cutting tool when the cutting plane is being obtained,
wherein weakened areas (19) are defined in said second guide (1),
said second guide (1) being obtained by means of an additive manufacturing process from a 3D model, which is generated from computerised axial tomographies (CAT) or magnetic resonance, with the particularity that the second guide (1) includes, on the lower sector (7) thereof, an additional protrusion (8) with the corresponding axial hole thereof for the passage of the seventh Kirschner wire (3‴),
such that, once the different wires have been implanted with the second guide (1),
said second guide (1) can be removed,
said second pair of parallel Kirschner wires (3') and said third pair of parallel wires (3") can be removed,
and the pair of upper and parallel Kirschner wires (3) can act as insertion means for the first surgical guide (9) through the pair of upper protrusions (14) of said first surgical guide (9), such that the open end of the lateral groove (11) is initially arranged facing the seventh Kirschner wire (3‴), such that when the rotation of the lower section (5) of the bone with respect to the upper section (5') of the bone is carried out on the cutting plane (10), the seventh wire (3‴) will be moved together with said lower section (5), to a point wherein the bottom (12) of said lateral groove (11) stops against the seventh Kirschner wire (3‴).

2. The kit according to claim 1, wherein the first surgical guide (9) further includes two additional protrusions (13) with axial through holes, for guiding a pair of additional Kirschner wires (3^{iv}) parallel to each other, intended to cross through both sections of the bone (5'-5), once both sections of the bone (5'-5) are in the correction position thereof, determined by immobilisation means of both sections during the implantation process of an osteosynthesis plate (20), staples or intramedullary nails between both bone sections.

3. The kit according to claim 1, wherein the protrusions (13-14-2-2'-2"-8) of both the first surgical guide (9) and the second guide (1) include markings with the insertion order of the Kirschner wires (3-3'-3"-3"').

4. The kit according to claims 1 and 3, wherein the Kirschner wires (3-3'-3ʺ-3‴-3^{iv}) include markings of the protrusion wherein they must be inserted and the depth at which they must be inserted.

## Patentansprüche

1. Kit bestehend aus:
einer ersten Bohrschablone (9) für Derotationsosteotomie-Operationen mit einer Rotation auf einer Schnittebene eines unteren Abschnitts (5) eines Knochens in Bezug auf den oberen Abschnitt (5') des Knochens und einer zweiten Führungsschablone (1),
die erste Bohrschablone (9) besteht aus einem Führungskörper, der aus einem personalisierten 3D-Modell gewonnen wurde, das mittels computergestützter Axialtomographie (CAT) oder Magnetresonanz des zu operierenden Knochens erzeugt wurde, wobei der Führungskörper eine längliche Konfiguration aufweist mit einer Oberfläche, die auf dem Knochen aufliegt und den physikalischen Merkmalen dieses Knochens entspricht, mit der Besonderheit, dass der genannte Führungskörper (9) mindestens zwei obere Vorsprünge (14) aufweist, die mit Durchgangslöchern versehen sind, für ein Paar von oberen und parallelen Kirschnerdrähten (3) zur Stabilisierung der Führung auf dem oberen Abschnitt (5') des Knochens, ein Führungskörper, der in Übereinstimmung mit seinem unterem Ende eine seitliche Nut (11) aufweist, die verlängert und an einem Ende offen ist, wobei die genannte seitliche Nut (11) als Bewegungsführung und als Anschlag bei der Winkelbewegung der Rotation des unteren Abschnitts (5) des Knochens auf der Schnittebene (10) in Bezug auf den oberen Abschnitt (5') des Knochens, für einen siebten Kirschnerdraht (3‴) vorgesehen ist, der in den unteren Abschnitt (5) des Knochens implantiert werden kann.
**Dadurch gekennzeichnet, dass**:
die genannte zweite Führungsschablone (1) aus einem Stück mit einer länglichen Konfiguration erhalten wird, worin drei Vorsprungpaare (2-2'-2") mit axialen Bohrungen vorhanden sind, um jeweils ein Paar von parallelen Kirschnerdrähten (3-3'-3"), zwei mal zwei, zu beherbergen;
die drei Vorsprungpaare (2-2'-2") der genannten zweiten Führungsschablone (1) bestehen aus:
zwei oberen Vorsprüngen (2) zur Führung des Paares der oberen und parallelen Kirschnerdrähte (3), um die Osteotomieschablone (1) während der Operation in der geeigneten Position zu halten;
zwei mittleren Vorsprüngen (2') zum Führen eines zweiten Paares von parallelen Kirschnerdrähten (3'), wobei das zweite Paar paralleler Kirschnerdrähte (3') konfiguriert ist, um als Führung für das Schneidwerkzeug zu fungieren, wenn die Schnittebene während der Osteotomie erhalten wird; und
zwei unteren Vorsprüngen (2") zum Führen eines dritten Paares paralleler Drähte (3"), die als Anschlag für das Schneidwerkzeug dienen sollen, wenn die Schnittebene erreicht wird;
wobei in der zweiten Führungsschablone (1) geschwächte Bereiche (19) vorhanden sind,
die genannte zweite Führungsschablone (1) wird mittels eines additiven Herstellungsverfahrens aus einem 3D-Modell gewonnen, welches mithilfe der computergestützten Axialtomographie (CAT) oder der Magnetresonanz erzeugt wird, mit der Besonderheit, dass die zweite Führungsschablone (1) auf ihrem unteren Abschnitt (7) einen zusätzlichen Vorsprung (8) mit der entsprechenden axialen Bohrung für den Durchgang des siebten Kirschnerdrahts (3‴) aufweist,
so dass nach der Implantation der verschiedenen Drähte mit der zweiten Führungsschablone (1),
die genannte zweite Führungsschablone (1) entfernt werden kann,
das genannte zweite Paar paralleler Kirschnerdrähte (3') sowie das gennante dritte Paar der parallelen Drähte (3") können entfernt werden
und das Paar der oberen und parallelen Kirschnerdrähte (3) kann als Einführmittel für die erste Bohrschablone (9) durch das Paar der oberen Vorsprünge (14) der genannten ersten Bohrschablone (9) fungieren, sodass das offene Ende der seitlichen Nut (11) zunächst dem siebten Kirschnerdraht (3‴) zugewandt ist, wenn also die Rotation des unteren Knochenabschnitts (5) in Bezug auf den oberen Abschnitt (5') des Knochens auf der Schnittebene (10) durchgeführt wird, wird der siebte Draht (3‴) zusammen mit dem unteren Abschnitt (5) zu einem Punkt bewegt, an dem das Ende (12) der genannten seitlichen Nut (11) gegen den siebten Kirschnerdraht (3‴) anschlägt.

2. Kit gemäß Anspruch 1, worin die erste Bohrschablone (9) weiterhin zwei zusätzliche Vorsprünge (13) mit axialen Durchgangslöchern zum Führen eines Paares zusätzlicher paralleler Kirschnerdrähte (3^{iv}) aufweist, die dazu bestimmt sind, beide Abschnitte des Knochens (5'-5) zu durchqueren, nachdem sich beide Abschnitte des Knochens (5'-5) in ihrer korrigierten Position befinden, die durch Blockierungsmittel beider Abschnitte während des Implantationsprozesses einer Osteosyntheseplatte (20) mit Klammern oder Marknägeln zwischen beiden Knochenabschnitten bestimmt wird.

3. Kit gemäß Anspruch 1, worin die Vorsprünge (13-14-2-2'-2"-8) sowohl der ersten Bohrschablone (9) als auch der zweiten Führungsschablone (1) Markierungen mit der Einfügereihenfolge der Kirschnerdrähte (3-3'-3‴) enthalten.

4. Kit gemäß den Ansprüchen 1 und 3, worin
die Kirschnerdrähte (3-3'-3ʺ-3‴-3^{iv}) Markierungen des Vorsprungs, in den sie eingeführt werden müssen, sowie der Einführtiefe enthalten, in der sie eingeführt werden müssen.

## Revendications

1. Kit comprenant :
un premier guide chirurgical (9) pour les opérations d'ostéotomie de dérotation avec rotation sur un plan de coupe d'une section inférieure (5) d'un os par rapport à une section supérieure (5') de l'os, et un deuxième guide (1),
le premier guide chirurgical (9) étant constitué d'un corps de guidage obtenu à partir d'un modèle 3D personnalisé généré au moyen de tomographie axiale informatisée (CAT) ou de résonance magnétique de l'os à opérer, le corps de guidage ayant une configuration allongée, avec une surface reposant sur l'os complémentaire aux caractéristiques physiques de celui-ci, avec la particularité que le corps de guidage mentionné (9) présente au moins deux saillies supérieures (14), équipées de trous traversants, pour une paire de broches de Kirschner supérieures et parallèles (3) pour la stabilisation du guide sur la section supérieure (5') de l'os, un corps de guidage qui présente, en correspondance avec l'extrémité inférieure de celui-ci, une rainure latérale (11), allongée et ouverte sur l'une des extrémités de celui-ci, ladite rainure latérale (11) agissant comme guide de déplacement et comme butée dans le mouvement angulaire en rotation de la partie inférieure (5) de l'os sur le plan de coupe (10) par rapport à la section supérieure (5') de l'os pour une septième broche de Kirschner (3‴) qui peut être implantée dans la section inférieure (5) de l'os ;
**caractérisé en ce que** :
ledit deuxième guide (1) est obtenu en une seule pièce, avec une configuration allongée dans laquelle trois paires de saillies (2-2'-2") sont définies avec des trous axiaux de manière à ce que chacune d'elles loge une paire de broches de Kirschner parallèles (3-3'-3"), deux par deux ;
les trois paires de saillies (2-2'-2'') dudit deuxième guide (1) constituées de :
deux saillies supérieures (2) pour le guidage de la paire de broches de Kirschner supérieures et parallèles (3) pour le maintien du guide d'ostéotomie (1) dans la position appropriée durant l'opération ;
deux saillies intermédiaires (2') pour le guidage d'une deuxième paire de broches de Kirschner parallèles (3'), ladite deuxième paire de broches de Kirschner parallèles (3') étant configurée pour agir comme moyen de guidage pour l'outil de coupe lorsque le plan de coupe est obtenu durant l'ostéotomie ; et
deux saillies inférieures (2") pour le guidage d'une troisième paire de broches parallèles (3"), destinées à agir comme butée pour l'outil de coupe lorsque le plan de coupe est obtenu,
dans lequel des zones affaiblies (19) sont définies dans ledit deuxième guide (1),
ledit deuxième guide (1) étant obtenu au moyen d'un processus de fabrication additive à partir d'un modèle 3D, qui est généré à partir de tomographies axiales informatisées (CAT) ou de résonance magnétique, avec la particularité que le deuxième guide (1) comprend, sur le secteur inférieur (7) de celui-ci, une saillie supplémentaire (8) avec le trou axial correspondant de celui-ci pour le passage de la septième broche de Kirschner (3'''),
de sorte que, une fois que les différentes broches ont été implantées avec le deuxième guide (1),
ledit deuxième guide (1) peut être retiré,
ladite deuxième paire de broches de Kirschner parallèles (3') et ladite troisième paire de broches parallèles (3") peuvent être retirées,
et la paire de broches de Kirschner supérieures et parallèles (3) peut servir de moyen d'insertion pour le premier guide chirurgical (9) à travers la paire de saillies supérieures (14) dudit premier guide chirurgical (9), de telle sorte que l'extrémité ouverte de la rainure latérale (11) est initialement agencée faisant face à la septième broche de Kirschner (3'''), de telle sorte que lorsque la rotation de la partie inférieure (5) de l'os par rapport à la partie supérieure (5') de l'os est effectuée sur le plan de coupe (10), la septième broche (3''') sera déplacée avec ladite section inférieure (5), jusqu'à un point dans lequel le fond (12) de ladite rainure latérale (11) vient en butée contre la septième broche de Kirschner (3''').

2. Kit selon la revendication 1, dans lequel le premier guide chirurgical (9) comprend en outre deux saillies supplémentaires (13) avec des trous traversants axiaux, pour le guidage d'une paire de broches de Kirschner supplémentaires (3^{iv}) parallèles entre elles, destinées à se croiser à travers les deux sections de l'os (5'-5), une fois que les deux sections de l'os (5'-5) sont dans la position de correction de celui-ci, déterminée par des moyens d'immobilisation des deux sections durant le processus d'implantation d'une plaque d'ostéosynthèse (20), d'agrafes ou de clous centromédullaires entre les deux sections osseuses.

3. Kit selon la revendication 1, dans lequel les saillies (13-14-2-2'-2"-8) du premier guide chirurgical (9) et du deuxième guide (1) comprennent des marquages avec l'ordre d'insertion des broches de Kirschner (3-3'-3ʺ-3‴).

4. Kit selon les revendications 1 et 3, dans lequel
les broches de Kirschner (3-3'-3"-3‴-3^{iv}) comprennent des marquages de la saillie dans laquelle elles doivent être insérées et de la profondeur à laquelle elles doivent être insérées.
